# EUROPEAN PATENT APPLICATION

(11) **EP 4 721 784 A1**
(43) Date of publication of application: **08.04.2026**
(21) Application number: 23938873.9
(22) Date of filing: 31.05.2023
(51) Int. Cl.: A61M 5/178

(54) **INJECTION MECHANISM CAPABLE OF ADJUSTING INJECTION DOSE, AND INJECTION SYRINGE**

(71) Applicant: C C Biotechnology Corporation, Tainan City, Taiwan 70955 (TW)
(72) Inventor: YEH, Chin-Min, Tainan City Taiwan (CN); WANG, Chih-Wei, Tainan City Taiwan (CN)
(74) Representative: Melchior, Robin
(86) International application number: PCT/CN2023/097540
(87) International publication number: WO 2024/243882

(57) **Abstract**

An injection mechanism (1) capable of adjusting injection dose, and an injection syringe. In the injection mechanism (1), an injection push rod (80), and an injection dose adjustment assembly (1a) and a clutch control assembly (1b), which are connected to the injection push rod (80), are mounted in a housing (10); and in the injection dose adjustment assembly (1a), a tube (20) can be rotated by means of a dose adjustment knob (30) to drive a driving ring (50), such that an elastic assembly (40) generates an elastic force to drive the injection push rod (80).

## Description

### Field of the Invention

The present invention relates to a syringe, and especially an injection mechanism and a syringe capable of adjusting injection dose.

### Description of Related Art

Based on the safety regulations for liquid medicament injection, the conventional syringes already have dose metering injection functions. That is, after the dose metering type syringe is attached to a medicine container, a push rod behind the syringe is pushed to push a piston in the medicine container to move a certain distance through a linkage of a driving mechanism mounted inside the syringe, and then a predetermined dose of liquid medicament is output through a needle attached to a front end of the medicine container.

In conventional dose metering type syringes, injection mechanisms of some syringes have structures for controlling dose. After the injection mechanism is attached to a medicine container, it controls dose of each injection and is locked after repeatable uses in predetermined times, thereby reminding that the entire syringe must be discarded to ensure the safety of the injection pen.

Although the aforementioned dose metering syringe can provide the function of outputting a fixed dose of liquid medicament, the injection dose usually needs to be adjusted according to the needs of different injection objects during the use of the syringe. The conventional dose metering syringe lacks the function of adjusting the injection dose, and it is difficult to meet the needs of different users.

In order to solve the problem of being unable to adjust the injection dose, some of the conventional dose metering syringes have an injection mechanism with function of adjusting injection dose, to meet the different injection dose requirements of different users. The injection mechanism is assembled with a medicine container via a cartridge connection device thereof and has an adjustable propulsion device mounted in the cartridge connection device and extending into the medicine container. A knob of the cartridge connection device can drive a push rod of the adjustable propulsion device to move backward to a predetermined scale. During injection, the push rod is pressed to output a predetermined dose of liquid medicine from the medicine container, so that the injection dose of the syringe can be adjusted according to the needs of different users.

In the aforementioned syringe, the injection mechanism with function of adjusting injection dose can be connected to a medicine container with liquid medicine, so the user can adjust the injection dose according to the doctor's prescription. However, during the injection process of the injection mechanism of the syringe, the user must press the injection push rod of the injection mechanism throughout the process, which is laborious to operate. Moreover, to perform injection action again, the injection mechanism must be manually reset, which is inconvenient in use.

### Content of the Invention

The technical problems to be solved by the present invention are: an injection mechanism and a syringe capable of adjusting injection dose, which solve the problem that the injection mechanism of the conventional syringe only provides a quantitative injection function for multiple equal dose injections, cannot meet the needs of different users for different injection doses, and the injection dose cannot be adjusted according to the doctor's prescription, and the operations of some injection mechanisms capable of adjusting injection dose are laborious and inconvenient.

The technical solution proposed by the present invention is to provide an injection mechanism capable of adjusting injection dose, which is configured to be attached to a medicine container. The injection mechanism comprises:
a housing being hollow;
an injection push rod mounted in the housing and able to be driven to move forward axially to the medicine container to perform an injection action;
an injection dose adjustment assembly connected to the injection push rod and comprising:
   a tube, with the injection push rod disposed therein, rotatably and movably mounted in the housing, and able to move forward and backward in the housing;
   a dose adjustment knob rotatably attached to a rear end of the housing, detachably connected to the tube, and able to rotate the tube when the dose adjustment knob is connected to the tube;
   a driving ring mounted in the tube and screwed with the injection push rod;
   an elastic assembly mounted in the tube, arranged between the driving ring and the tube, able to provide an elastic force to connect the tube and the dose adjustment knob, and able to be driven by the driving ring to generate an elastic potential energy; and
   a pressing component disposed at a rear end of the dose adjustment knob, the pressing component able to trigger the tube to separate from the dose adjustment knob to make the elastic assembly release the elastic potential energy to push the driving ring to drive the injection push rod to move forward spirally; and
a clutch control assembly mounted within a front end of the housing, able to limit the injection push rod when the dose adjustment knob rotates the tube, able to connect the tube when the tube is triggered to separate from the dose adjustment knob; the elastic assembly driving the tube to separate from the clutch control assembly for restoring after the injection action is completed by the injection push rod.

Another technical solution proposed by the present invention is to provide a syringe capable of adjusting injection dose comprising: an injection mechanism capable of adjusting injection dose as aforementioned and a cartridge sleeve. The cartridge sleeve is sleeved on said medicine container and attached to the front end of the housing.

The present invention, by means of the above-mentioned injection mechanism and the syringe with the injection mechanism, has at least the following beneficial effects when the injection action is performed after the injection mechanism is assembled with the medicine container:
1. Dose adjustment function: in the injection mechanism, the injection dose adjustment assembly in the housing is connected to the injection push rod of the injection mechanism. The tube is rotated by the dose adjustment knob and drives the driving ring to move, so that the elastic assembly generates the elastic potential energy. When the tube is triggered to separate from the dose adjustment knob by the pressing component, the elastic assembly releases the elastic potential energy to push the driving ring to drive the injection push rod to move forward spirally. Whereby the injection mechanism is capable of adjusting injection dose.
2. Repeatedly reusable: the injection mechanism comprises the clutch control assembly disposed in the front end of the housing. When the tube is rotated by the dose adjustment knob, the clutch control assembly can limit the injection push rod. When the tube is triggered to sperate from the dose adjustment knob, the clutch control assembly can connect to the tube. After the injection action of the injection push rod is completed, the elastic assembly drives the tube to separate from the clutch control assembly and to return to an original position. Whereby, the injection mechanism can adjust injection dose and perform the injection action repeatedly.
3. Labor-saving and ease of operation: in the injection mechanism, the injection dose adjustment assembly in the housing and the injection push rod of the injection mechanism are connected as mentioned above. The tube is rotated by the dose adjustment knob and drives the driving ring to move, so that the elastic assembly generates the elastic potential energy. When the tube is triggered to separate from the dose adjustment knob by the pressing component, the elastic assembly releases the elastic potential energy to the driving ring to push the injection push rod to move forward spirally. Whereby the injection mechanism is labor-saving and facilitates ease of operation.

The injection mechanism and the syringe in accordance with the present invention may further provide function of precisely adjusting injection dose. Wherein the housing has a scale window. Multiple dose scales are arranged around an outer peripheral surface of the tube. When the dose adjustment knob connects the tube, the dose adjustment knob is able to be rotated and to align any one of the dose scales of the tube with the scale window. When the dose adjustment knob is rotated to align one of the dose scales of the tube with the scale window, the elastic assembly is driven by the driving ring to generate said elastic potential energy corresponding to said dose scale, and the clutch control assembly limits the injection push rod. Therefore, the injection mechanism and the syringe can provide the function of precisely adjusting injection dose according to the doctor's prescription.

The injection mechanism and the syringe in accordance with the present invention may further provide a locking function. Wherein, in the injection mechanism, the injection push rod comprises a forward-spiral-guiding groove and a reverse-spiral-guiding groove formed in an outer peripheral surface of the injection push rod, and a stopping portion formed at a rear end of the injection push rod behind the driving ring. The driving ring has a forward-spiral portion therein connected to the forward-spiral-guiding groove via threaded connection. The clutch control assembly comprises a connecting component and a clutch control ring in the connecting component. The clutch control ring has a reverse-spiral portion therein. The reverse-spiral portion is connected to the reverse-spiral-guiding groove of the injection push rod via threaded connection. The clutch control ring is able to engage with and disengage from the tube being driven. The connecting component can limit the clutch control ring to be rotated reversely at a predetermined point. The connecting component has a forward-threaded-connection portion to connect the forward-spiral-guiding groove of the injection push rod via threaded connection. With such structural arrangement, when the liquid medicine in the medicine container is used up, the stopping portion at the rear end of the injection push rod blocks the rear end of the driving ring for limiting the injection push rod. When the liquid medicine in the medicine container is less than the predetermined injection dose and a distance between the stopping portion at the rear end of the injection push rod and the abutment ring behind the driving ring is less than a movement for the predetermined dose injection, the user rotates the dose adjustment knob to turn the tube to drive the driving ring to be limited by the stopping portion at the rear end of the injection push rod, the user cannot rotate the dose adjustment knob any further to a position for a larger injection dose, and it is locked.

### Description of the Drawings

Fig. 1 is a plan view of an embodiment of an injection mechanism capable of adjusting injection dose attached to a medicine container.
Fig. 2 is an exploded perspective view of the injection mechanism in Fig. 1.
Fig. 3 is an exploded perspective view of the injection mechanism in Fig. 1 viewed from another angle.
Fig. 4 is a perspective view in partial section of a housing of the injection mechanism in Figs. 2 and 3.
Fig. 5 is a perspective view in partial section of a tube of the injection mechanism in Figs. 2 and 3.
Fig. 6 is a perspective view in partial section of a dose adjustment knob of the injection mechanism in Figs. 2 and 3.
Figs. 7 and 8 are perspective views in partial sections of a driving ring of the injection mechanism in Figs. 2 and 3 viewed from different angles.
Figs. 9 and 10 are perspective views in partial sections of a clutch controlling ring of the injection mechanism in Figs. 2 and 3 viewed from different angles.
Fig. 11 is a perspective view in partial section of a connecting component of the injection mechanism in Figs. 2 and 3.
Fig. 12 is a perspective view (1) of an assembly with some components of the injection mechanism in Figs. 2 and 3.
Fig. 13 is a perspective view (2) of an assembly with some components of the injection mechanism in Figs. 2 and 3.
Fig. 14 is a cross-sectional view of the injection mechanism attached to the medicine container across line A-A in Fig. 1.
Fig. 15 is a cross-sectional view of the injection mechanism attached to the medicine container across line B-B in Fig. 1.
Fig. 16 is a cross-sectional view of the injection mechanism attached to the medicine container across line C-C in Fig. 1.
Fig. 17 is a cross-sectional view of the injection mechanism attached to the medicine container across line D-D in Fig. 1.
Fig. 18 is a perspective view of an embodiment of a syringe capable of adjusting injection dose in accordance with the present invention.
Fig. 19 is a partial exploded perspective view of the syringe in Fig. 18.
Fig. 20 is an operational reference scheme (1) of the syringe in Figs. 18 and 19.
Fig. 21 is an operational reference scheme (2) of the syringe in Figs. 18 and 19.
Fig. 22 is an operational reference scheme (3) of the syringe in Figs. 18 and 19.
Fig. 23 is an operational reference scheme (4) of the syringe in Figs. 18 and 19.
Fig. 24 is an operational perspective view (1) in partial section of the syringe in Figs. 18 and 19.
Fig. 25 is a partial perspective view of the syringe in Fig. 24.
Fig. 26 is an operational perspective view (2) in partial section of the syringe in Figs. 18 and 19.
Fig. 27 is an operational perspective view (3) in partial section of the syringe in Figs. 18 and 19.
Fig. 28 is a partial perspective view of the syringe in Figs. 26 and 27.
Fig. 29 is an operational perspective view (4) in partial section of the syringe in Figs. 18 and 19.
Fig. 30 is an operational perspective view (5) in partial section of the syringe in Figs. 18 and 19.

### Description of the Reference Numbers:

1: injection mechanism
L: central axis
E1: front end
E2: rear end
F: forward direction
1a: injection dose adjustment assembly
1b: clutch control assembly
10: housing
11: housing wall
12: containing space
13: scale window
14: front-end-connecting portion
141: annular groove
142: notch
143: axial guiding groove
144: locking indent
15: rear-end-connecting portion
151: front-blocking portion
152: rear-blocking portion
16: housing-wall-engaging tooth
20: tube
200: assembling space
21: dose scale marking set
211: exhaust icon
22: toothed portion
23: guiding block
24: alignment groove
25: movable guiding groove
26: abutment portion
27: front-positioning portion
28: rear-positioning portion
30: dose adjustment knob
300: attaching space
31: inner annular board
32: clutch tooth
40: elastic assembly
41: abutment ring
411: abutting portion
50: driving ring
51: forward-spiral portion
52: axial-guiding portion
53: protrusion
60: clutch control ring
61: reverse-spiral portion
62: elastic buckle
63: alignment tooth
70: connecting component
700: movable space
71: forward-threaded-connection portion
72: connecting rail
73: locking block
74: single oblique ratchet tooth
80: injection push rod
801: forward-spiral-guiding groove
802: reverse-spiral-guiding groove
803: front end
804: stopping portion
81: piston abutment
90: pressing component
2: medicine container
2A: piston
3: cartridge sleeve
31: needle-connecting-end portion
32: attached-end portion
33: cartridge window
4: protection cap
5: needle
6: needle shield set
6A: needle shield sleeve
6B: needle cap

### Description of Present Invention

The following describes the technical means adopted by the present invention to achieve the intended purpose of the invention in conjunction with the drawings and embodiments of the present invention.

The technical solutions of the present invention comprise an injection mechanism capable of adjusting injection dose and a syringe capable of adjusting injection dose having the injection mechanism. In conjunction with the drawings and illustrated embodiments, specific compositions and structures of the injection mechanism and the syringe of the present invention are respectively illustrated as follows.

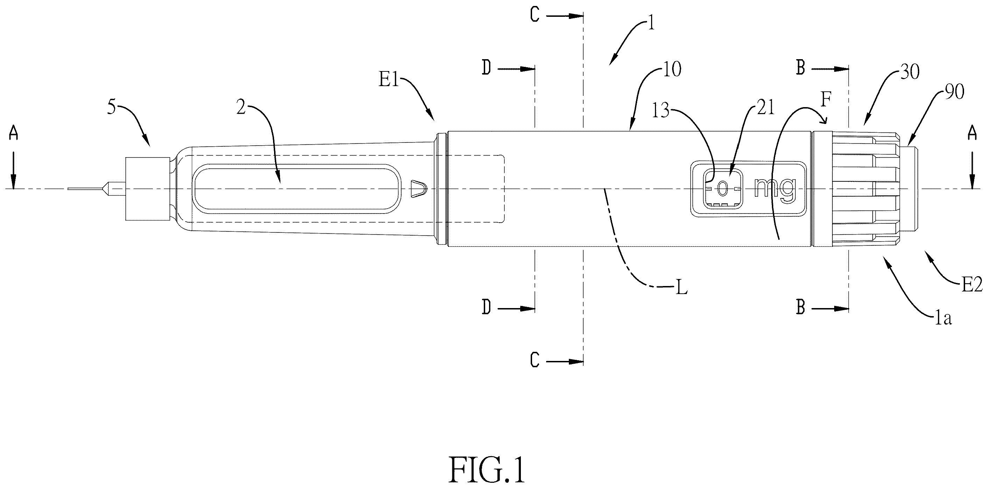

As shown in Fig. 1, an embodiment of the injection mechanism 1 is disclosed. A medicine container 2 may be directly or indirectly attached to a front end of the injection mechanism 1 with a needle 5 attached to a front end of the medicine container 2 to constitute a syringe capable of adjusting injection dose. In the foregoing, the medicine container 2 contains liquid medicine and a piston. The needle 5 extends through a plug at a front end of the medicine container and extends into the medicine container. When the piston in the medicine container 2 is pushed by the injection mechanism 1, the liquid medicine inside the medicine container 2 can be outputted through the needle 5 to perform an injection action.

In order to conveniently illustrate the embodiment of the injection mechanism 1 of the present invention, direction is defined firstly. Wherein, as shown in Fig. 1, it is defined that the injection mechanism 1 has a central axis L. A direction of the central axis L is defined as an axial direction, and a direction perpendicular to the central axis L is a radial direction. An end, along the central axis L, of the injection mechanism 1 configured to be attached to the medicine container is defined as a front end E1 thereof. The opposite end is defined as a rear end E2 of the injection mechanism 1. A clockwise rotation around the central axis L relative to the rear end E2 is defined as a forward direction F, on the other hand, the counterclockwise rotation is defined as a reverse direction.

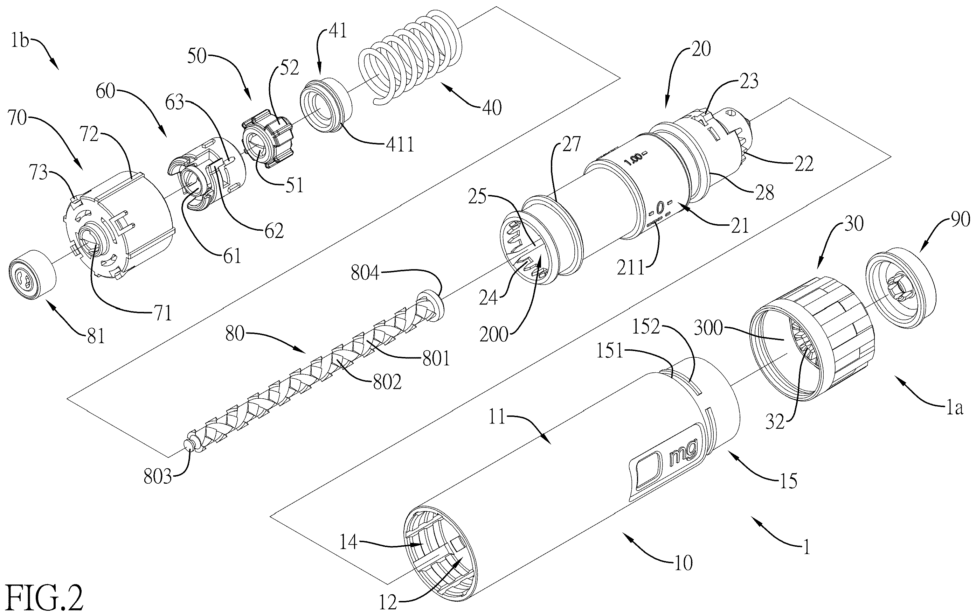

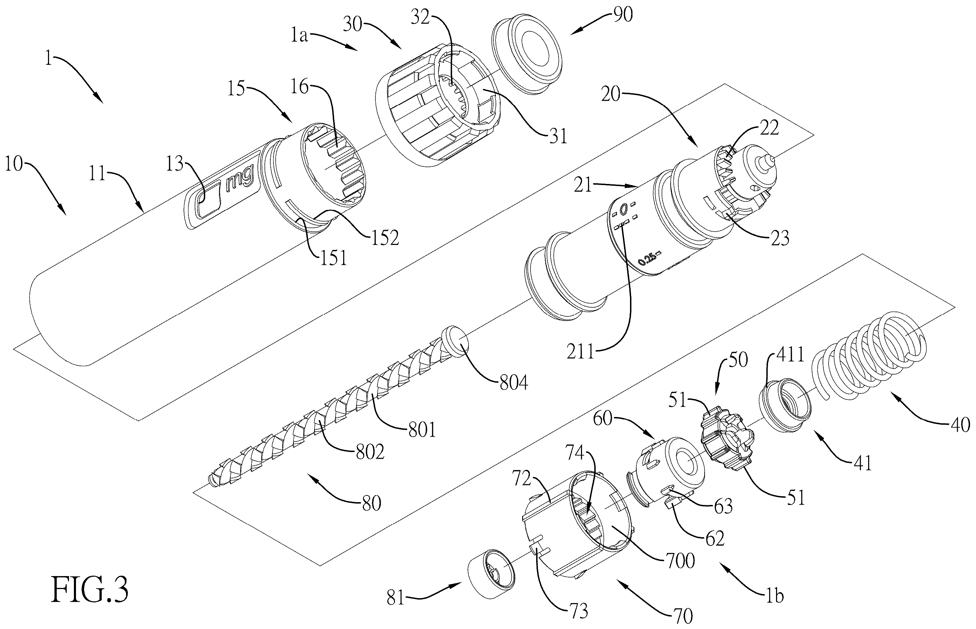

As shown in Figs. 2 and 3, the injection mechanism 1 comprises a housing 10, an injection push rod 80, an injection dose adjustment assembly 1a, and a clutch control assembly 1b. Details of the injection mechanism 1 are described as follows.

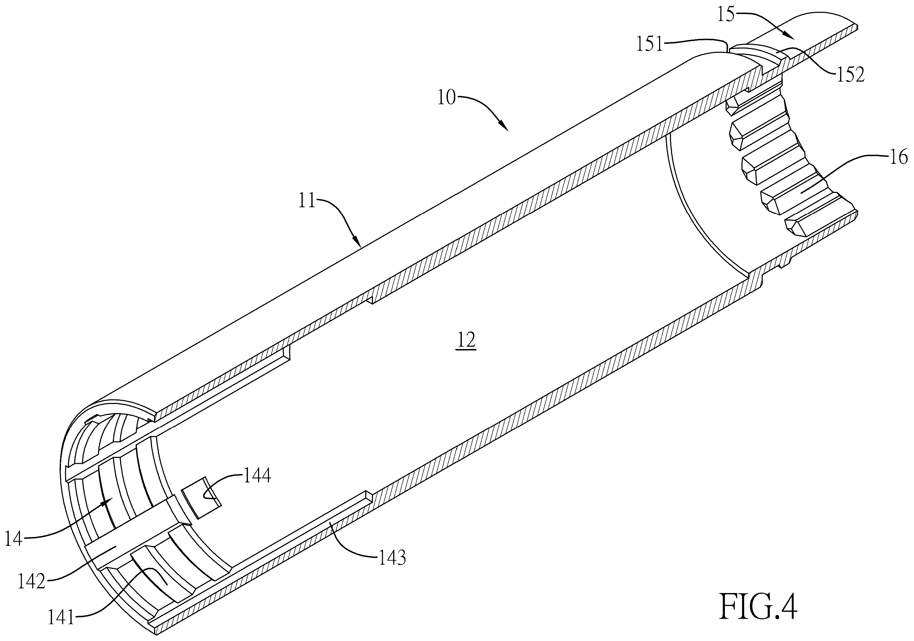

As shown in Figs. 2, 3, and 4, the housing 10 comprises a housing wall 11 and a containing space 12 in the housing wall 11 and formed therethrough along the central axis. The housing 10 has a scale window 13 formed in an outer peripheral surface of the housing wall 11 and radially communicating with the containing space 12.

In the embodiment as shown in Figs. 2, 3, and 4, the housing 10 has a front-end-connecting portion 14 at a front end thereof configured to be connected to a rear end of the medicine container 2. The housing 10 has a rear-end-connecting portion 15 at a rear end thereof and configured to be connected to the injection dose adjustment assembly 1a.

In the embodiment shown in Figs. 2, 3, and 4, the front-end-connecting portion 14 of the housing 10 has multiple annular grooves 141 formed in an inner peripheral surface of the housing wall 11 and spaced apart from one another in a front-to-back arrangement, multiple notches 142 parallel to the central axis, and multiple locking indents 144. The rear-end-connecting portion 15 has one or more rear-blocking portions 152 and a front-blocking portion 151 in front of and spaced apart from said rear-blocking portions 152. The rear-end-connecting portion 15 has multiple housing-wall-engaging teeth 16 formed at the inner peripheral surface of the housing wall 11 and arranged at equiangular intervals. The housing 10 has at least one axial guiding groove 143 formed in the inner peripheral surface of the housing wall 11 and extending backwardly from the front end.

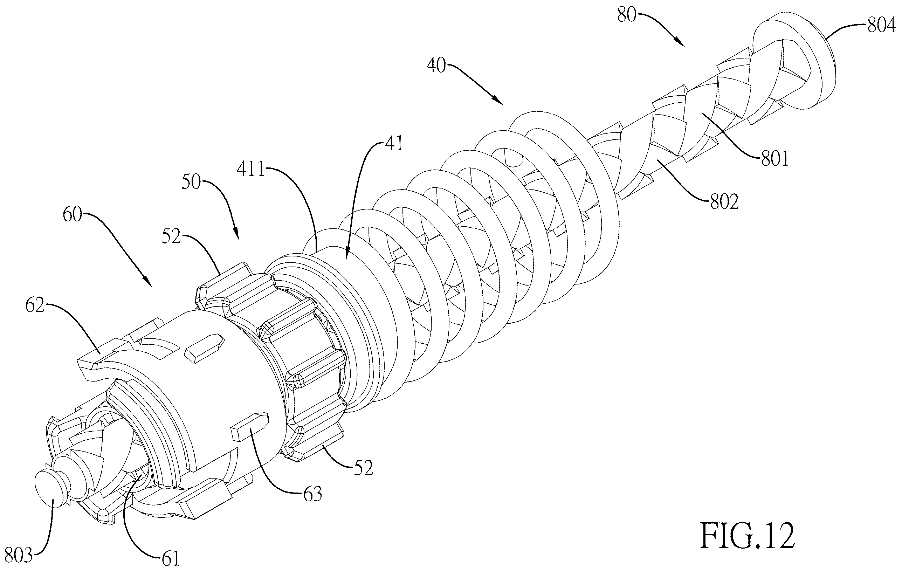

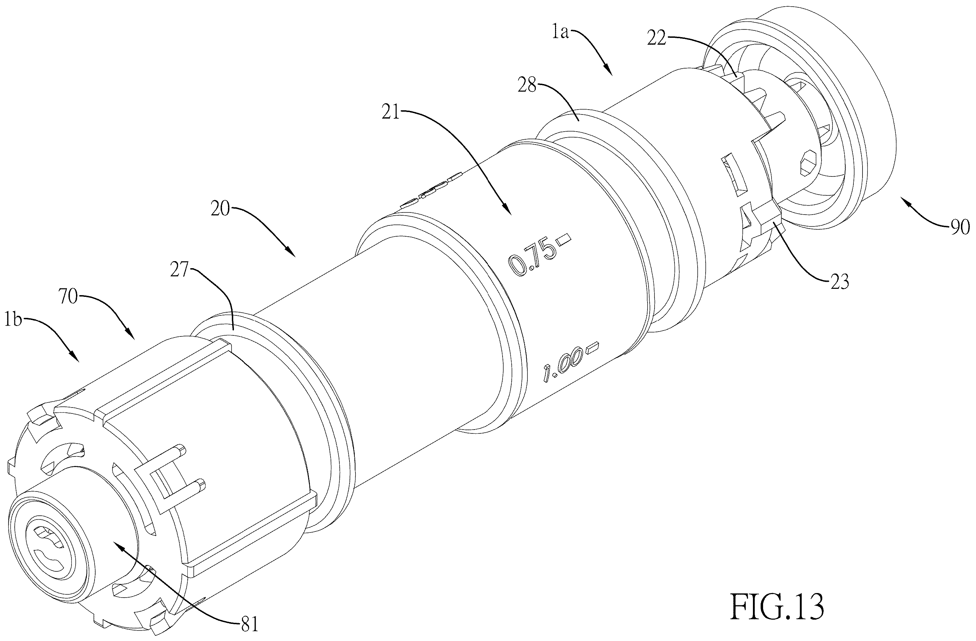

As shown in Figs. 2, 3, and 12 to 14, the injection push rod 80 is disposed in the housing 10 and is able to be driven to move forward along the central axis. The piston 2A in the medicine container 2 may be directly driven by the injection push rod 80 to perform an injection action or be driven by a piston abutment 81 combined with a front end 803 of the injection push rod 80 to perform an injection. In the embodiment, the injection push rod 80 has a forward-spiral-guiding groove 801 and a reverse-spiral-guiding groove 802 formed in an outer peripheral surface thereof. A pitch of the forward-spiral-guiding groove 801 is equal to a pitch of the reverse-spiral-guiding groove 802. The pitches of the forward-spiral-guiding groove 801 and the reverse-spiral-guiding groove 802 and a length of the injection push rod 80 are able to go with the injection dose adjustment assembly 1a and are designed according to an output dose of the medicine container 2 driven by the injection push rod 80. The injection push rod 80 has a stopping portion 804 at a rear end thereof to provide limitation and stopping functions. In the embodiment, the pitches of the forward-spiral-guiding groove 801 and the reverse-spiral-guiding groove 802 are the same. However, in other embodiments, the pitch of the forward-spiral-guiding groove 801 may not be equal to the pitch of the reverse-spiral-guiding groove 802 according to a requirement for adjusting speed or other requirements. It is not limited to this.

As shown in Figs. 2, 3, and 14, the injection dose adjustment assembly 1a is disposed in the housing 10 and is connected to the injection push rod 80. The front end of the injection push rod 80 extends out of a front end of the injection dose adjustment assembly 1a. The injection dose adjustment assembly may be manually adjusted to set multiple various dose values to generate elastic potential energies in various magnitudes correspondingly. When the injection dose adjustment assembly is triggered, the elastic potential energies drive the injection push rod 80 to move various distances. Whereby, the injection push rod 80 drives the medicine container to perform injections with various doses.

As shown in Figs. 2, 3, and 14, the injection dose adjustment assembly 1a comprises a tube 20, a dose adjustment knob 30, a driving ring 50, an elastic assembly 40, and a pressing component 90.

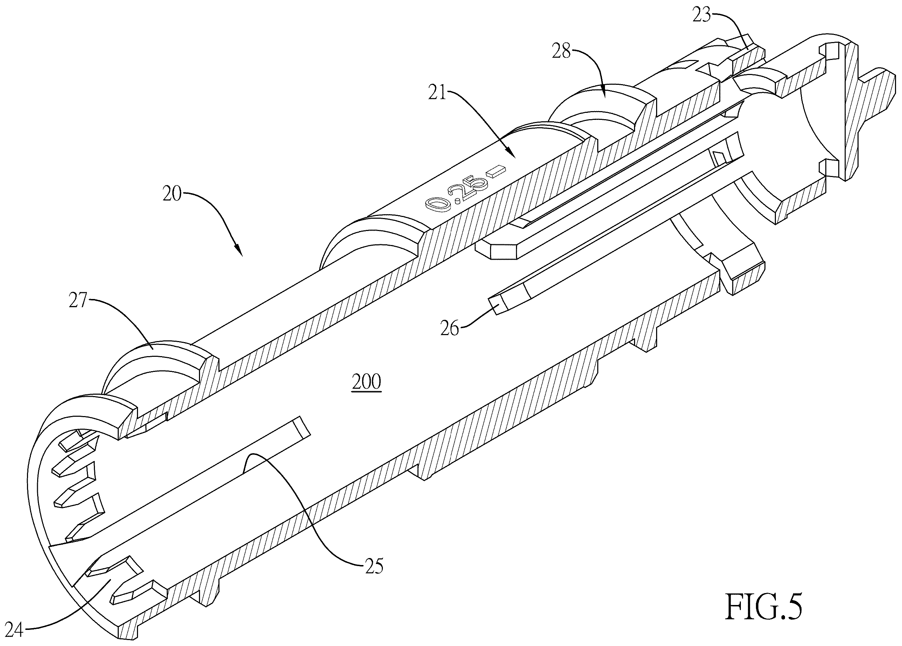

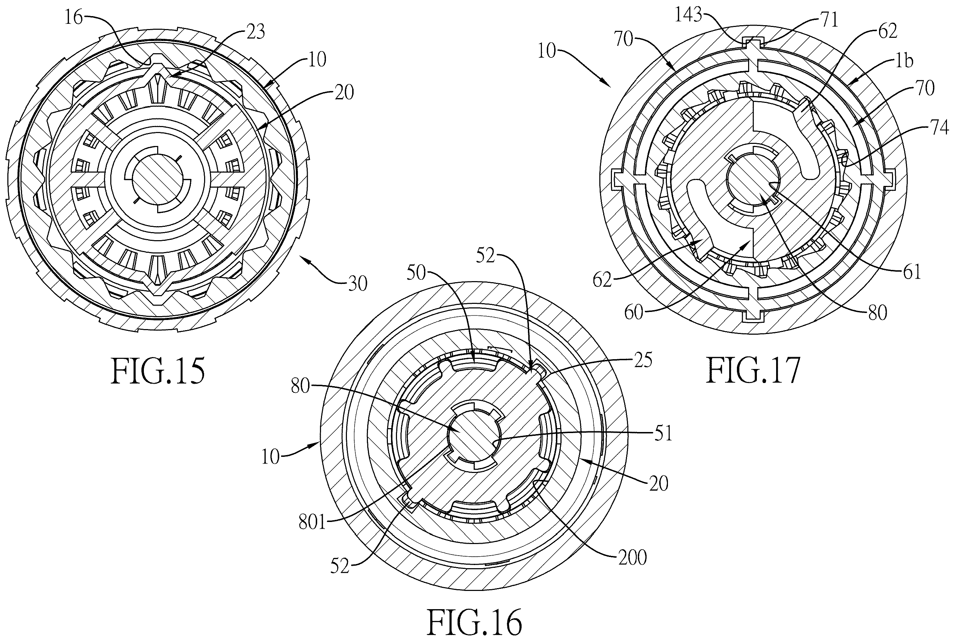

As shown in Figs. 2, 3, 5, 14, and 15, the tube 20 is rotatable, is movable along the central axis, and is mounted in the containing space 12 of the housing 10. The tube 20 has a dose scale marking set 21 formed at an outer peripheral surface thereof. The dose scale marking set 21 has multiple dose scales. The dose scales are set according to injection dosages of doctor's usual prescriptions or according to injection dosages of doctor' specific prescriptions. In the embodiment, the dose scale marking set 21 comprises multiple dose scales such as "-0- (mg)", "0.25- (mg)", "0.5- (mg)", "0.75- (mg)", and "1.0- (mg)". The multiple dose scales are arranged around the outer peripheral surface of the tube 20 at predetermined positions, but it is not limited to this.

The dose scale marking set 21 may further comprise an exhaust icon 211 which is "--- -" icon in the present embodiment. The "--- -" icon is adjacent to a position of the scale of "-0- (mg)", representing an indication to remove air from the medicine container. The dose scales and exhaust icon 211 of the dose scale marking set 21 may be selectively shown in the scale window 13 of the housing 10 by rotating the tube 20.

The tube 20 has an assembling space 200 formed therein. The tube 20 has multiple alignment grooves 24 and at least one movable guiding groove 25 formed in an inner peripheral surface of a front end of the tube 20 and extending backwardly. At least one abutment portion 26 is formed at a rear section of the assembling space 200 of the tube 20. Multiple toothed portions 22 are formed at the outer peripheral surface of a rear end of the tube 20 and extend backwardly along the central axis. At least one guiding block 23 is located in front of the toothed portions 22 and radially protrudes. The guiding block 23 is able to engage with the housing-wall-engaging teeth 16 in the rear end of the housing 10. The injection push rod 80 extends into the assembling space 200 of the tube 20.

A front-positioning portion 27 is formed at the outer peripheral surface of the tube 20 at a front section thereof. A rear-positioning portion 28 is formed at the outer peripheral surface of the tube 20 at a rear section thereof. The rear-positioning portion 28 is located between the dose scale marking set 21 and the guiding block 23 to limit a movable range of the tube 20 forward and backward along the central axis.

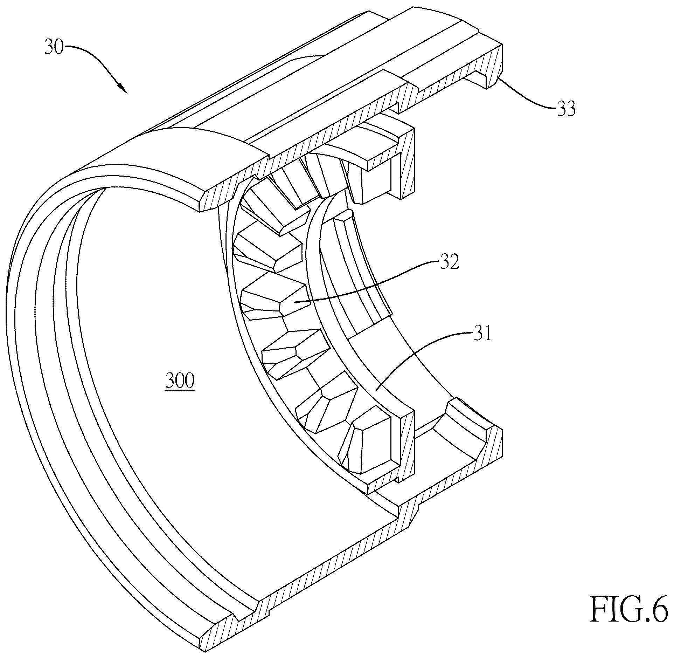

As shown in Figs. 2, 3, 6, and 14, the dose adjustment knob 30 is rotatably sleeved on the rear-end-connecting portion 15 of the housing 10 and is connected to the tube 20. The dose adjustment knob 30 is able to drive the tube 20 to align one of the dose scales or the exhaust icon 211 of the dose scale marking set 21 with the scale window 13 of the housing 10. The dose adjustment knob 30 has an attaching space 300 formed therein, an inner annular board 31 formed at a rear section of the attaching space 300, and multiple clutch teeth 32 arranged around a front surface of the inner annular board 31. The clutch teeth 32 of the dose adjustment knob 30 are able to engage with the toothed portions 22 at the rear end of the tube 20. When the tube 20 is moved forward, the clutch teeth 32 are separated from the toothed portions 22.

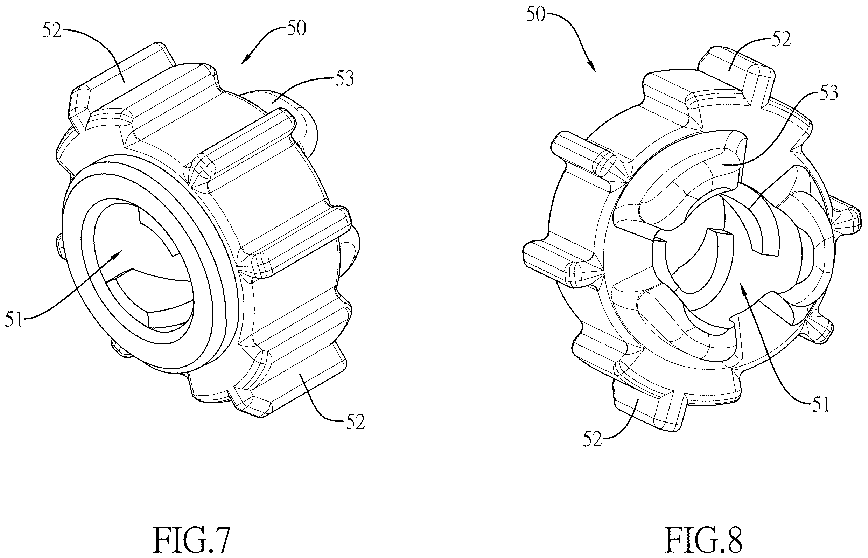

As shown in Figs. 2, 3, 7, 8, and 14, the driving ring 50 is mounted in the assembling space 200 of the tube 20 and is screwed with the injection push rod 80. In the present embodiment, the driving ring 50 has a forward-spiral portion 51 formed therein. The forward-spiral portion 51 is connected to the forward-spiral-guiding groove 801 of the injection push rod 80 via threaded connection. At least one axial-guiding portion 52 is formed at an outer peripheral surface of the driving ring 50. The axial-guiding portion 52 is mounted in the movable guiding groove 25 of the tube 20. Whereby, the driving ring 50 can move forward and backward inside the tube 20 along the axial direction. The driving ring 50 can be rotated around the injection push rod 80 with the tube 20.

As shown in Figs. 2, 3, and 14, the elastic assembly 40 is mounted in the assembling space 200 of the tube 20 and is operated to generate elastic potential energy. In the embodiment, the elastic assembly 40 is a compression spring. The elastic assembly 40 is sleeved around the injection push rod 80, and is pre-compressed between the driving ring 50 and the abutment portion 26 in the assembling space 200 of the tube 20. The elastic assembly 40 is pre-compressed to generate a push force to push the tube 20 to move backward, so that the tube 20 and the dose adjustment knob 30 are in a connected condition.

With reference to Figs. 2, 3, 7, 8, and 14, in the above, a rear end of the elastic assembly 40 abuts against the abutment portion 26 in the tube 20. An abutment ring 41 is mounted to a front end of the elastic assembly 40. The injection push rod 80 extends through the abutment ring 41. The abutment ring 41 is movable along the injection push rod 80. The elastic assembly 40 is connected to the driving ring 50 via the abutment ring 41. The abutment ring 41 has an abutting portion 411 formed at an outer peripheral surface thereof to provide a portion to be abutted by the front end of the elastic assembly 40. The driving ring 50 has one or multiple protrusions 53 formed at a rear end thereof and is rotatably mounted in the abutment ring 41 via the protrusions 53, so the driving ring 50 abuts against a front end of the abutment ring 41 and is rotatable.

As shown in Figs. 2, 3, 12, and 14, the pressing component 90 is disposed at the rear end of the housing 10, is moveable forward and backward, and is mounted to a rear end of the dose adjustment knob 30. The pressing component 90 can be pressed forward to push the tube 20 to move forward, and to separate the toothed portions 22 of the tube 20 from the clutch teeth 32 of the dose adjustment knob 30.

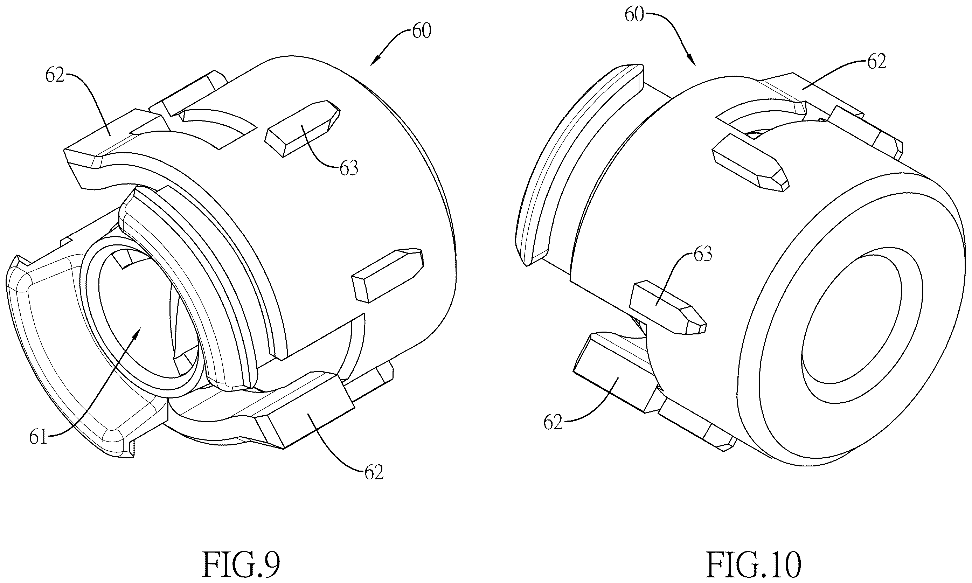

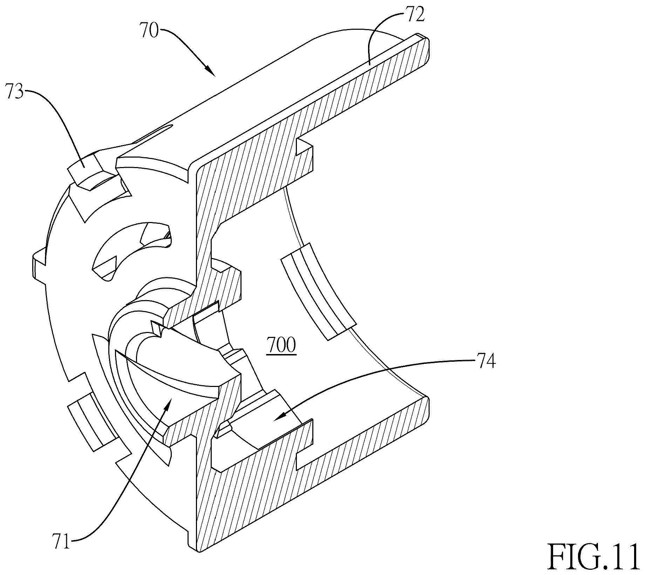

As shown in Figs. 2, 3, 9 to 11, and 14, the clutch control assembly 1b is mounted in a front of the housing 10 and is disposed in front of the tube 20 and the driving ring 50. The clutch control assembly 1b is able to engage with and disengage from the tube 20. When one of the dose scales of the tube 20 is rotated to the scale window 13 by the dose adjustment knob 30, the clutch control assembly 1b can limit a position of the injection push rod 80. And when the tube 20 is triggered to disengage from the dose adjustment knob 30, the clutch control assembly 1b can be connected to the tube 20. After the injection push rod 80 completes the injection action, the tube 20 is driven to disengage from the clutch control assembly 1b by the elastic assembly 40. In the embodiment, the clutch control assembly 1b comprises a clutch control ring 60 and a connecting component 70.

As shown in Figs. 2, 3, 9 to 11, and 14, the clutch control ring 60 is disposed in front of the tube 20 and the driving ring 50. The clutch control ring 60 is able to engage with and disengage from the tube 20 being operated. The clutch control ring 60 has a reverse-spiral portion 61 formed therein. The reverse-spiral portion 61 and the forward-spiral portion 51 of the driving ring 50 are with spirals in opposite directions. The injection push rod 80 is screwed with and disposed in the clutch control ring 60. The reverse-spiral portion 61 and the reverse-spiral-guiding groove 802 of the injection push rod 80 are in a threaded engagement. The clutch control ring 60 has multiple alignment teeth 63 and multiple elastic buckles 62 formed at an outer peripheral surface thereof. The alignment teeth of the clutch control ring 60 are able to engage with the alignment grooves 24 in the front end of the tube 20, whereby the clutch control ring 60 can be rotated with the tube 20.

As shown in Figs. 2, 3, 11, and 14, the connecting component 70 is mounted in the front-end-connecting portion 14 of the housing 10 and is located in front of the tube 20. The front-positioning portion 27 and the rear-positioning portion 28 of the tube 20 are located between the connecting component 70 and a rear end portion of the housing 10. The connecting component 70 has a central hole and a movable space 700 behind the central hole. A forward-threaded-connection portion 71 is formed in the central hole. The injection push rod 80 is screwed with the connecting component 70. The piston abutment 81 disposed at the front end of the injection push rod 80 is located in front of the connecting component 70. The forward-threaded-connection portion 71 is connected to the forward-spiral-guiding groove 801 of the injection push rod 80 via a threaded connection. The connecting component 70 has multiple single oblique ratchet teeth 74 arranged around an inner peripheral surface of the movable space 700. A front end of the clutch control ring 60 is rotatably mounted in the movable space 700 of the connecting component 70. The elastic buckles 62 of the clutch control ring 60 are able to engage with the single oblique ratchet teeth 74 and the clutch control ring 60 can only be driven to rotate reversely at a predetermined point.

As shown in Figs. 2, 3, 11, 14, and 17, the connecting component 70 has at least one connecting rail 72 and multiple locking blocks 73 formed at an outer peripheral surface thereof. The connecting component 70 is mounted in the front-end-connecting portion 14 of the housing 10. The connecting rail 72 is aligned with the axial guiding groove 143 of the housing 10, and the locking blocks 73 are engaged with the locking indents 144 of the housing 10, whereby the connecting component 70 is fixed in the housing 10.

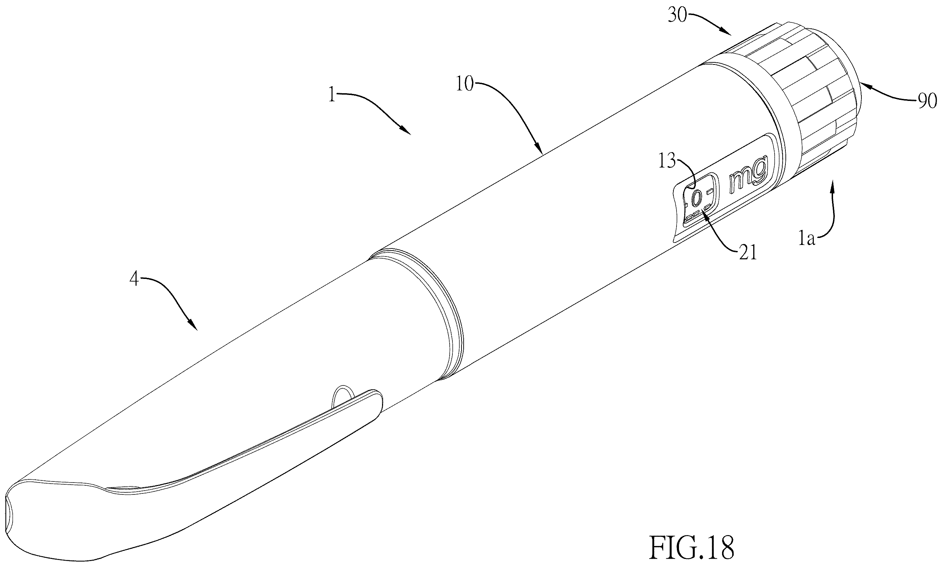

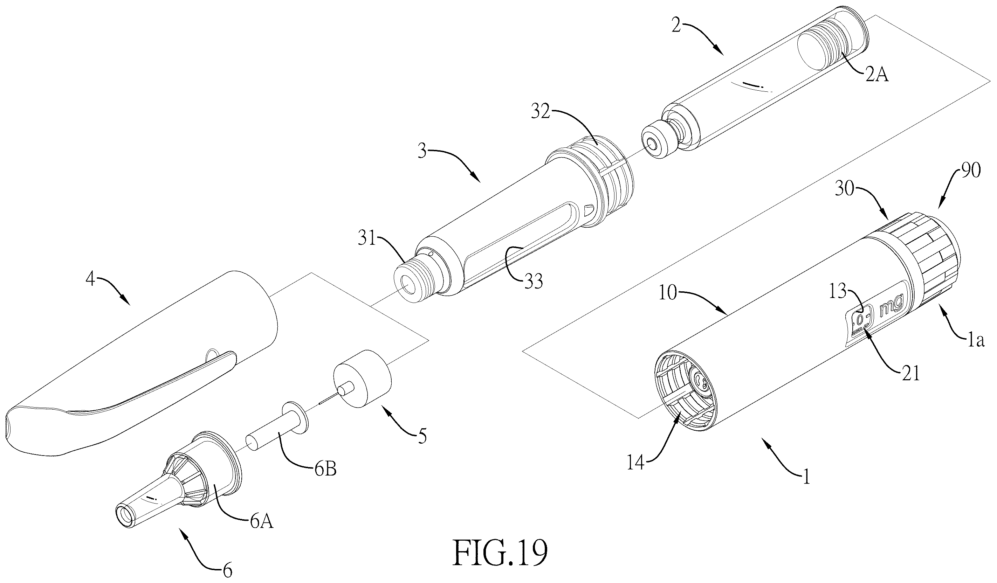

As shown in Figs. 18 and 19, an embodiment of the syringe capable of adjusting dose in accordance with the present invention is disclosed. As shown in the drawings, the syringe comprises said injection mechanism 1 and a cartridge sleeve 3. The injection mechanism 1 is described as the foregoing, and details thereof are omitted.

As shown in Fig. 19, the cartridge sleeve 3 is sleeved on the medicine container 3, and the cartridge sleeve 3 facilitates the medicine container 3 to be attached to the front end of the housing 10 of the injection mechanism 1. The piston abutment 81 assembled to the front end of the injection push rod 80 extends into the medicine container 2 and abuts against a rear end of the piston 2A. A front end of the cartridge sleeve 3 is detachably connected to the needle 5. The needle 5 extends into the medicine container 2 through the front end of the cartridge sleeve 3.

As shown in Figs. 19, 14, and 4, the cartridge sleeve 3 has an attached-end portion 32 at a rear end thereof. An outer peripheral surface of the attached-end portion 32 corresponds to the front-end-connecting portion 14 of the housing 10 at the inner peripheral surface of the housing wall 11 in shape. That is, the attached-end portion 32 of the cartridge sleeve 3 has annular protrusions corresponding to the annular grooves 141 of the front-end-connecting portion 14 and elongated protrusions corresponding to the notches 142. The attached-end portion 32 at a rear end of the cartridge sleeve 3 can be detachably mounted in the front-end-connecting portion 14 of the housing 10. After assembled, the attached-end portion 32 at the rear end of the cartridge sleeve 3 can be firmly fixed in the front-end-connecting portion 14 of the housing 10.

As shown in Fig. 19, the cartridge sleeve 3 has a needle-connecting-end portion 31 at the front end thereof. The needle 5 is detachably attached to the needle-connecting-end portion 31. The needle 5 extends through the front end of the cartridge sleeve 3 and is inserted into the medicine container 2. The cartridge sleeve 3 has one or more cartridge windows 33 formed in a peripheral wall thereof. The user can observe the status of the liquid medicine in the medicine container 2 through the cartridge windows 33.

As shown in Fig. 19, a protection cap 4 may be sleeved on the cartridge sleeve 3. The attached-end portion 32 at the rear end of the cartridge sleeve 3 extends out of a rear end of the protection cap 4. The cartridge sleeve 3 and the medicine container 3 within the cartridge sleeve 3 can be protected by the protection cap 4. When the cartridge sleeve 3 is mounted in the protection cap 4, the user can attach the medicine container 3 to the cartridge sleeve 3 inside the protection cap 4 and holds the protection cap 4 to insert the attached-end portion 43 at the rear end of the cartridge sleeve 3, extending out of the rear end of the protection cap 4, into the front-end-connecting portion 14 of the housing 10 of the injection mechanism 1. When the syringe is a pen-type product, the protection cap 4 may be formed as a pen cap.

As shown in Fig. 19, the needle 5 may be surrounded by a needle shield set 6. The needle shield set 6 comprises a needle cap 6B and a needle shield sleeve 6A. The needle cap is sleeved on the needle. The needle shield sleeve 6A is sleeved on the needle cap 6B to protect the needle 5. The needle shield sleeve 6A may be directly sleeved on the needle 5 at the front end of the cartridge sleeve 3. The needle shield sleeve 6A is a safety auxiliary tool for removing the needle 5, so the user can detach the needle 5 from the front end of the cartridge sleeve 3 safely.

Regarding usage of the injection mechanism 1 and the syringe comprising the injection mechanism 1, for convenience of explaining the present invention, the syringe with the injection mechanism 1 is taken as the subject of explanation, as follows.

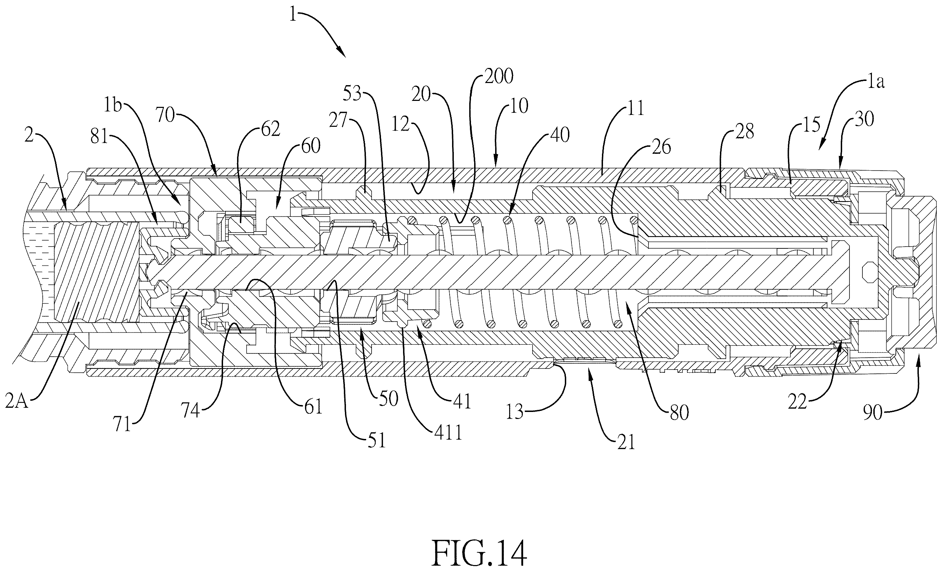

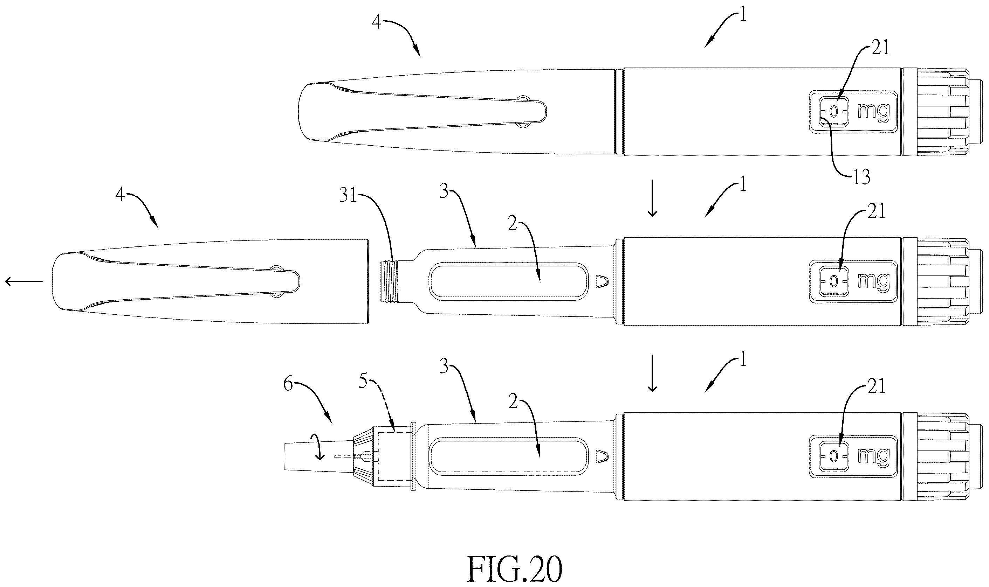

As shown in Figs. 20 and 14, in an initial condition before use, the medicine container 2 is attached to the front end of the injection mechanism 1 via the cartridge sleeve 3. The protection cap 4 is sleeved on the cartridge sleeve 3 with the medicine container 2. The scale of the"0.- (mg)" of the dose scale marking set 21 of the tube 20 is shown in the scale window 13 of the housing 10. Before use, the protection cap 4 is removed, the needle shield set 6 with the needle 5 therein is attached to the front end of the cartridge sleeve 3, and then the needle shield set 6 on the needle 5 is removed. The needle 5 is attached to the needle-connecting-end portion 31 at the front end of the cartridge sleeve 3. The needle 5 extends through the front end of the cartridge sleeve 3 and is inserted into and fluidly communicates with the medicine container 2.

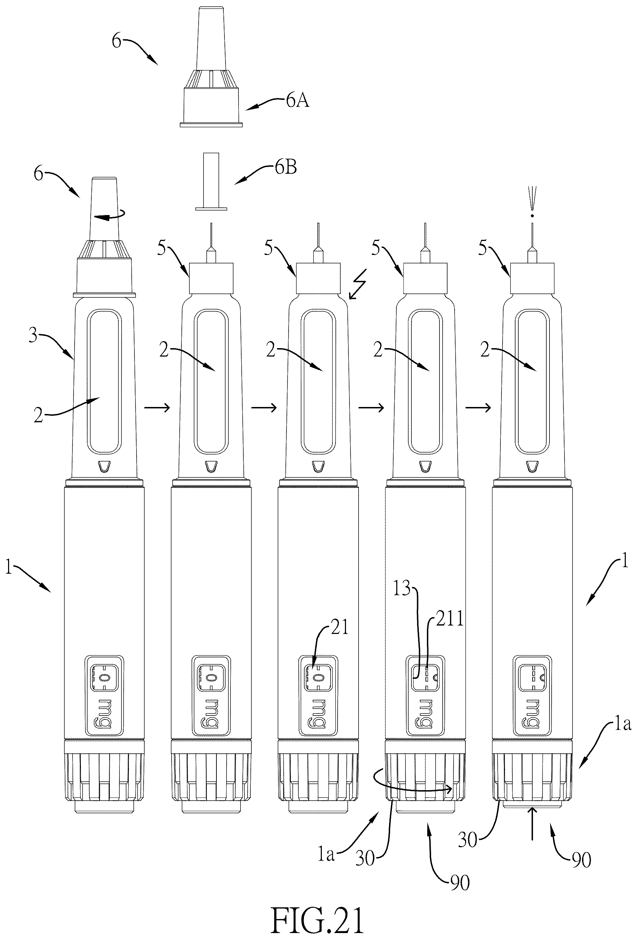

Generally, after the medicine container 2 with the liquid medicine is sealed, some bubbles may remain in the medicine container 2. As shown in Fig. 21, to prevent the bubbles from being injected into a human body, before injection, the front end of the medicine container 2 with the needle 5 is pointed up, and the cartridge sleeve 3 sleeved on the medicine container 2 is flicked several times, letting the bubbles move to the top of the medicine container 2. The dose adjustment knob 30 of the injection mechanism 1 is rotated to drive the tube 20 to turn the dose scale marking set 21 to align the exhaust icon 211 of "--- -" with the scale window 13 of the housing 10 from the scale of "0.- (mg)" of the tube 20. The pressing component 90 is pushed to drive the injection push rod 80 in the injection mechanism 1 to push the piston 2A in the medicine container 2 while the front end of the medicine container 2 with the needle 5 points up to exhaust the bubbles in the medicine container 2 totally.

In the above, the process that the dose adjustment knob 30 of the injection mechanism 1 drives the exhaust icon 211 "--- -" of the dose scale marking set 21 of the tube 20 to the scale window 13 of the housing 10 and then the pressing component 90 is pressed to drive the injection push rod 80 to execute exhausting the bubbles in the medicine container 2, which is the same as the injection action described below. Therefore, when the bubbles are exhausted, the action of the injection mechanism 1 is described below.

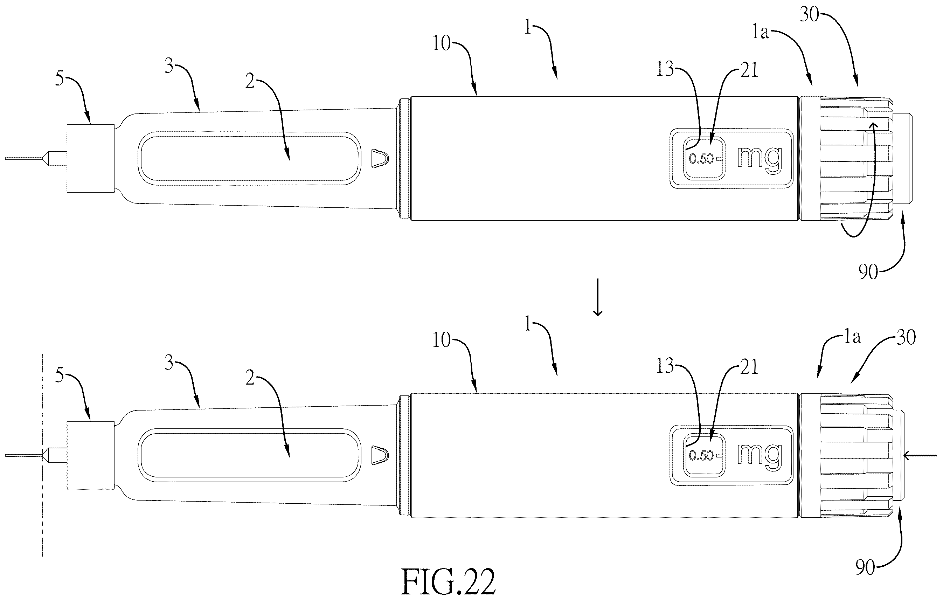

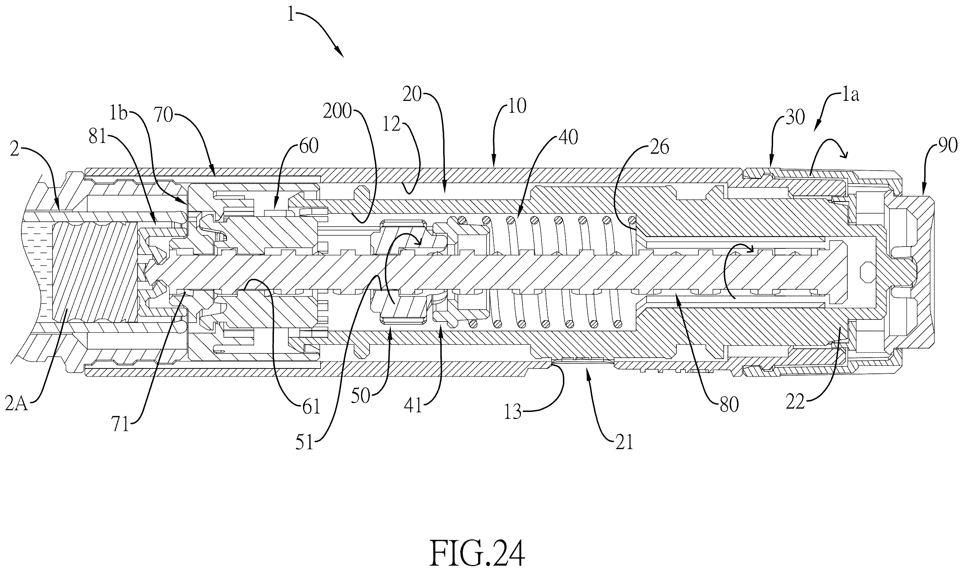

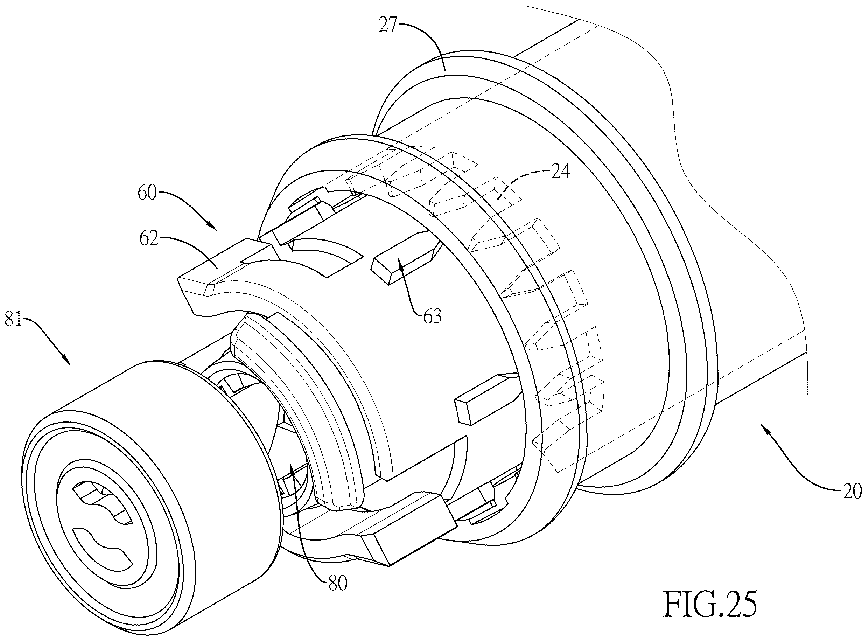

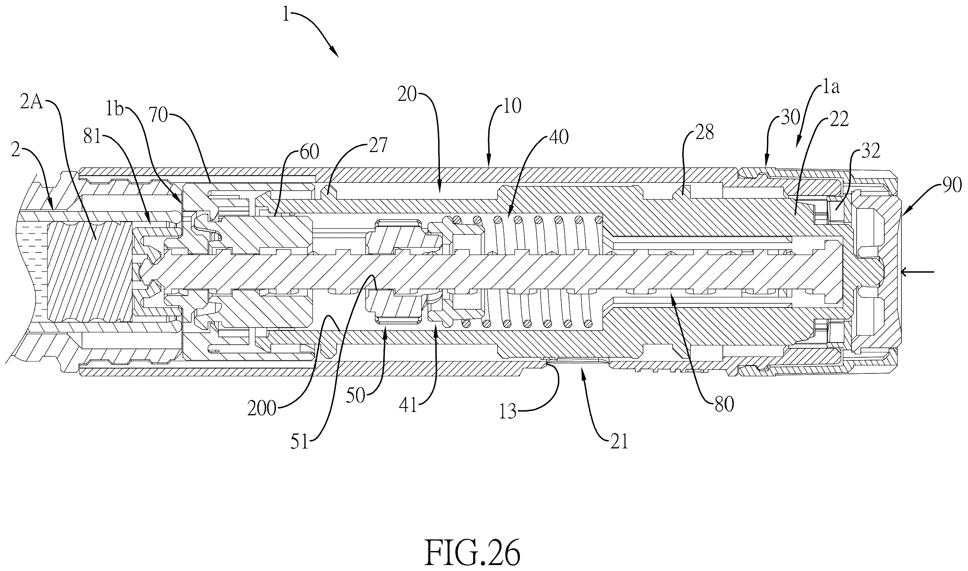

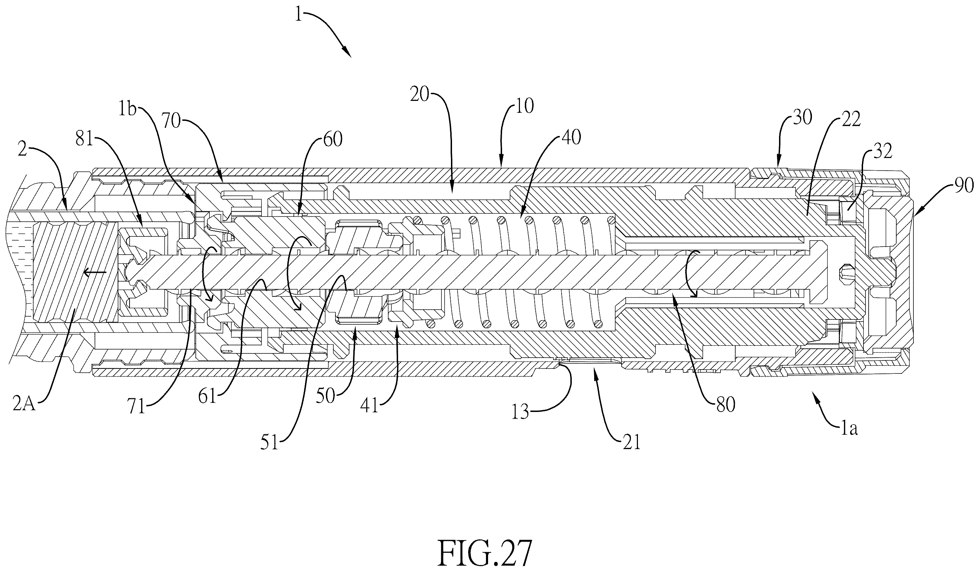

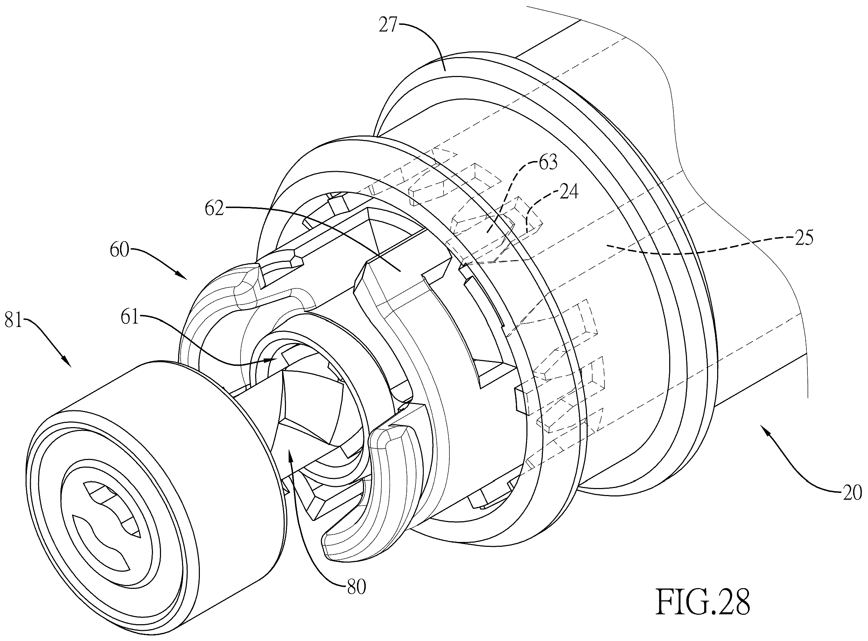

As shown in Figs. 22 and 24 to 28, during injection, firstly, the dose adjustment knob 30 is rotated to align a predetermined dose scale of the dose scale marking set 21 of the tube 20 with the scale window 13. For example, the predetermined injection dose is 0.75mg, and the dose adjustment knob 30 is rotated to drive the dose scale marking set 21 of the tube 20 to align the scale of "0.75- (mg)", from the scale of "0.- (mg)", with the scale window 13. Then the needle 5 is inserted into an injection region of the human body, and the pressing component 90 is pressed to perform the injection action. The steps of setting the injection dose by rotating and the injection action are as follows.

During the process that the predetermined dose scale of the dose scale marking set 21 of the tube 20 is moved to the scale window 13 of the housing 10 by the dose adjustment knob 30, the injection push rod 80 is limited in position by the connecting component 70 fixed in the housing 10 and the clutch control ring 60 restricted in the connecting component 70. The clutch teeth 32 of the dose adjustment knob 30 engage with the toothed portions 22 at the rear end of the tube 20. The driving ring 50 in the tube 20 extends into the movable guiding groove 25 in the tube 20 via the axial-guiding portion 52. So when the dose adjustment knob 30 is forwardly rotated to align the scale of "0.75- (mg)" with the scale window 13 from the scale of "0.- (mg)", the tube 20 is forwardly rotated with the dose adjustment knob 30 and drives the driving ring 50 to rotate backwardly along the forward-spiral-guiding groove 801 of the injection push rod 80 to a position corresponding to the injection dose. The abutment ring 41 compresses the elastic assembly 40 to store elastic potential energy.

When the pressing component 90 is pressed forwardly, the pressing component 90 pushes the tube 20 to move forward, the toothed portions 22 at the rear end of the tube 20 are separated from the clutch teeth 32 of the dose adjustment knob 30, and the alignment grooves 24 at the front end of the tube 20 are engaged with the alignment teeth 63 of the clutch control ring 60 in front thereof. Then, under the elastic potential energy of the elastic assembly 40, the driving ring 50 is driven to move forward and stopped when contacting the clutch control ring 60. At the same time, the threaded connection of the forward-spiral portion 51 of the driving ring 50 and the forward-spiral-guiding groove 801 of the injection push rod 80 with the threaded connection of the forward-threaded-connection portion 71 of the connecting component 70 and the forward-spiral-guiding groove 801 of the injection push rod 80, the injection push rod 80 is spirally moved forward under the elastic potential energy. Whereby the piston abutment 81 at the front end of the injection push rod 80 pushes the piston 2A in the medicine container 2 to move forward, and the predetermined dose of the liquid medicine is injected into the human body from the medicine container 2 via the needle 5.

As shown in Figs. 22 to 28, in the abovementioned spiral propulsion of the injection push rod 80, with the threaded connection of the reverse-spiral portion 61 of the clutch control ring 60 and the reverse-spiral-guiding groove 802 of the injection push rod 80 and the engagement of the alignment grooves 24 at the front end of the tube 20 and the alignment teeth 63 of the clutch control ring 60 in front thereof, the injection push rod 80 is pushed to spirally move forward by the elastic potential energy and drives the clutch control ring 60, the tube 20, and the driving ring 50 in the tube 20 to rotate reversely at the same time until the dose scale of "0.- (mg)" of the tube 20 is rotated back to the scale window 13 of the housing 10.

After the injection is completed, the driving ring 50 is kept in the position of contacting the rear end of the clutch control ring 60. The tube 20 is pushed backwardly by the elastic force of the elastic assembly 40, as a result, the alignment grooves 24 at the front end of the tube 20 are separated from the alignment teeth 63 of the clutch control ring 60 in front thereof, and the toothed portions 22 at the rear end of the tube 20 are re-engaged with the clutch teeth 32 of the dose adjustment knob 30 to complete the injection action once. After the injection action is completed, the user sleeves the needle shield sleeve 6A on the needle 5. With the needle shield sleeve 6A as a safety auxiliary tool for removing the needle 5, the needle 5 is safely removed from the front end of the cartridge sleeve 3. The protection cap 4 is sleeved on the cartridge sleeve 3 for next injection.

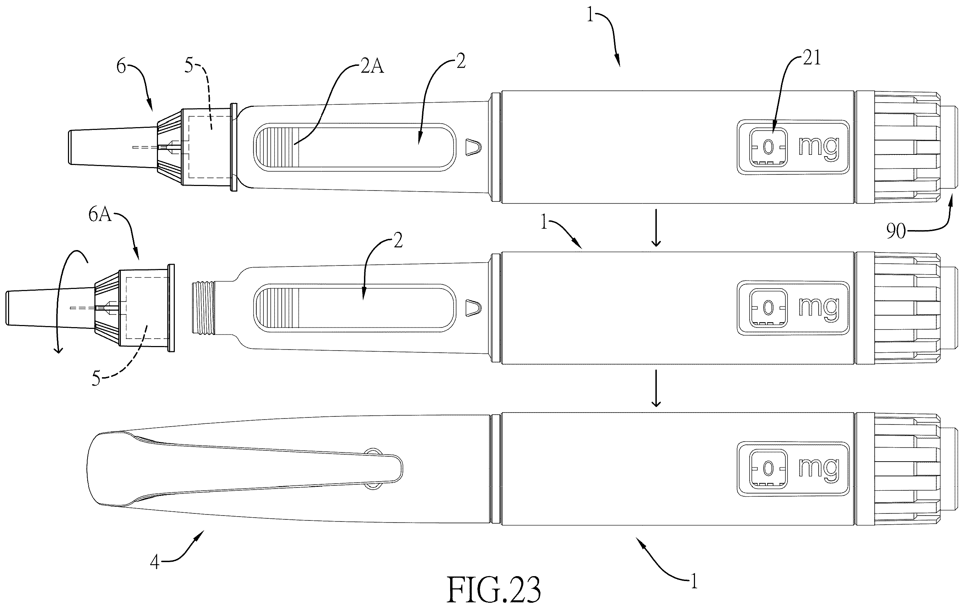

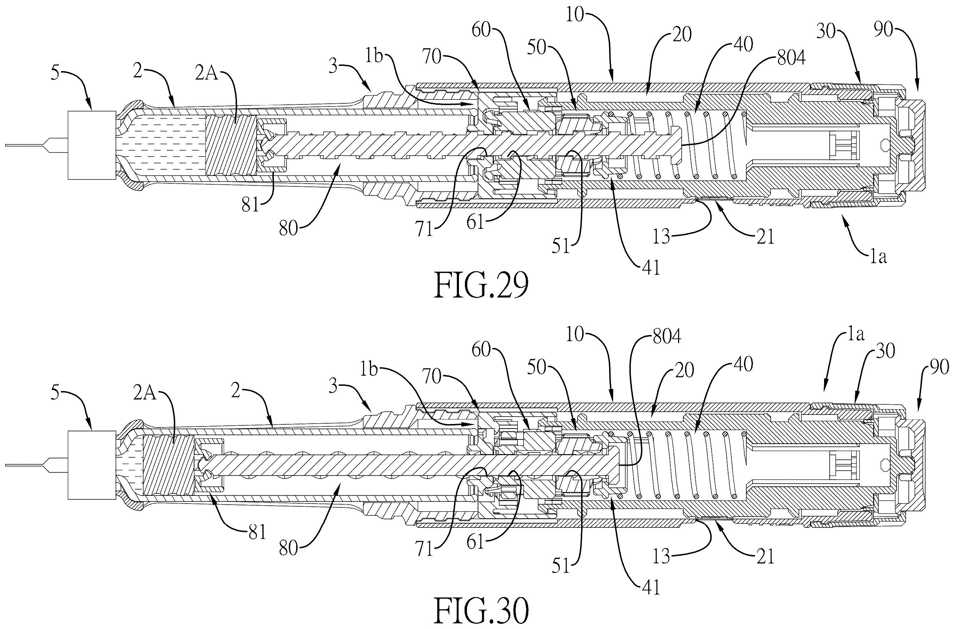

As shown in Figs. 23 and 30, proceed with the foregoing operation until the liquid medicine in the medicine container 2 is used up. The injection mechanism 1 uses the connecting component 70 fixed in the housing 10 to limit the clutch control ring 60 to be driven to rotate reversely at a predetermined point and the threaded connection of the clutch control ring 60 and the reverse-spiral-guiding groove 802 of the injection push rod 80. When the dose adjustment knob 30 is rotated, the injection push rod 80 is limited and cannot be rotated. Therefore, if the liquid medicine remaining in the medicine container 2 is less than a predetermined dose, as shown in Fig. 29, a distance between the stopping portion 804 at the rear end of the injection push rod 80 and the abutment ring 41 behind the driving ring 50 is less than an injection stroke movement for the predetermined dose. For example, when the predetermined dose of the liquid medicine is 0.75 mg, and the remaining liquid medicine in the medicine container 2 is 0.5 mg, the distance between the stopping portion 804 and the abutment ring 41 behind the driving ring 50 only corresponds to the movement for injection dose of 0.5 mg. When the user rotates the dose adjustment knob 30 to rotate the dose scale marking set 21 of the tube 20 to align the scale window 13 with the scale of "0.5- (mg)" from the scale of "0.- (mg)", the abutment ring 41 behind the driving ring 50 contacts the stopping portion 804 at the rear end of the injection push rod 80 to be locked by the stopping portion 804, so the user cannot rotate the dose adjustment knob 30 any further to align the scale of "0.75- (mg)" with the scale window 13.

Similarly, as shown in Figs. 23 and 30, when the liquid medicine in the medicine container 2 is used up, the rear side of the abutment ring 41 behind the driving ring 50 is also locked by the stopping portion 804 at the rear end of the injection push rod 80. The user cannot further rotate the dose adjustment knob 30. When the liquid medicine in the medicine container 2 is used up, after the needle shield sleeve 6A is sleeved on the needle 5 and the needle 5 is safely removed from the front end of the cartridge sleeve 3, the protection cap 4 is sleeved on the cartridge sleeve 3, and the syringe is discarded together with the empty medicine container 2. The syringe is used for limited times to ensure safety of the syringe.

The above descriptions are only the preferred embodiments of the present invention and do not limit the present invention in any form. Although the present invention has been disclosed as above in preferred embodiments, it is not intended to limit the present invention. Anyone familiar with the professional technology, without departing from the scope of the technical solution of the present invention, can make use of the technical content disclosed above to make slight changes or modification into equivalent embodiments with equivalent changes, but any content that does not depart from the technical solution of the present invention is based on the present invention. Any simple modifications, equivalent changes and modifications made to the above embodiments by technical essence still fall within the scope of the technical solutions of the present invention.

## Claims

1. An injection mechanism (1) capable of adjusting injection dose, configured to be attached to a medicine container (2), wherein
the injection mechanism (1) comprises:
a housing (10) being hollow;
an injection push rod (80) mounted in the housing (10) and able to be driven to move forward axially to the medicine container (2) to perform an injection action;
an injection dose adjustment assembly (1a) connected to the injection push rod (80) and comprising:
a tube (20), with the injection push rod (80) disposed therein, rotatably and movably mounted in the housing (10), and able to move forward and backward in the housing (10);
a dose adjustment knob (30) rotatably attached to a rear end of the housing (10), detachably connected to the tube (20), and able to rotate the tube (20) when the dose adjustment knob (30) is connected to the tube (20);
a driving ring (50) mounted in the tube (20) and screwed with the injection push rod (80);
an elastic assembly (40) mounted in the tube (20), arranged between the driving ring (50) and the tube (20), able to provide an elastic force to connect the tube (20) and the dose adjustment knob (30), and when the dose adjustment knob (30) rotates the tube (20), the elastic assembly (40) driven by the driving ring (50) to generate an elastic potential energy; and
a pressing component (90) disposed at a rear end of the dose adjustment knob (30), the pressing component (90) able to trigger the tube (20) to separate from the dose adjustment knob (30) to make the elastic assembly (40) release the elastic potential energy to push the driving ring (50) to drive the injection push rod (80) to move forward spirally; and
a clutch control assembly (1b) mounted within a front end of the housing (10), able to limit the injection push rod (80) when the dose adjustment knob (30) rotates the tube (20), able to connect the tube (20) when the tube (20) is triggered to separate from the dose adjustment knob (30); the elastic assembly (40) driving the tube (20) to separate from the clutch control assembly (1b) for restoring after the injection action is completed by the injection push rod (80).

2. The injection mechanism (1) capable of adjusting injection dose as claimed in claim 1, wherein
the housing (10) has a scale window (13); multiple dose scales are arranged around an outer peripheral surface of the tube (20); the dose adjustment knob (30) is rotatable to align any one of the dose scales of the tube (20) with the scale window (13) when the dose adjustment knob (30) connects the tube (20);
when the dose adjustment knob (30) rotates the tube (20) and aligns one of the dose scales of the tube (20) with the scale window (13), the elastic assembly (40) is driven by the driving ring (50) to generate said elastic potential energy corresponding to said dose scale and the clutch control assembly (1b) limits a position of the injection push rod (80).

3. The injection mechanism (1) capable of adjusting injection dose as claimed in claim 2, wherein, the injection push rod (80) has a forward-spiral-guiding groove (801) and a reverse-spiral-guiding groove (802) formed in an outer peripheral surface of the injection push rod (80); and a stopping portion (804) is formed at a rear end of the injection push rod (80) behind the driving ring (50);
the driving ring (50) has a forward-spiral portion (51) connected to the forward-spiral-guiding groove (801) via threaded connection;
the clutch control assembly (1b) comprises:
a clutch control ring (60) mounted in front of the tube (20) and the driving ring (50) and having a reverse-spiral portion (61); the reverse-spiral portion (61) and the forward-spiral portion (51) of the driving ring (50) having spirals in opposite directions; the injection push rod (80) screwed within the clutch control ring (60); the reverse-spiral portion (61) connected to the reverse-spiral-guiding groove (802) via threaded connection; the clutch control ring (60) able to engage with and disengage from the tube (20) being driven; and
a connecting component (70) mounted in the housing (10) at the front end of the housing (10), located in front of the clutch control ring (60), and limiting the clutch control ring (60) to be rotated reversely at a predetermined point; the connecting component (70) having a central hole and a forward-threaded-connection portion (71) inside the central hole; the injection push rod (80) screwed within the connecting component (70), and the forward-threaded-connection portion (71) connected to the forward-spiral-guiding groove (801) via threaded connection.

4. The injection mechanism (1) capable of adjusting injection dose as claimed in claim 3, wherein, a pitch of the forward-spiral-guiding groove (801) is equal to a pitch of the reverse-spiral-guiding groove (802).

5. The injection mechanism (1) capable of adjusting injection dose as claimed in claim 3, wherein, a pitch of the forward-spiral-guiding groove (801) is unequal to a pitch of the reverse-spiral-guiding groove (802).

6. The injection mechanism (1) capable of adjusting injection dose as claimed in claim 3, wherein, multiple alignment grooves (24) and at least one movable guiding groove (25) extending backwardly are formed in an inner peripheral surface of a front end of the tube (20);
at least one axial-guiding portion (52) is formed at an outer peripheral surface of the driving ring (50); said axial-guiding portion (52) is mounted in said movable guiding groove (25) of the tube (20); the driving ring (50) is movable forward and backward in the tube (20);
multiple alignment teeth (63) and multiple elastic buckles (62) are formed at an outer peripheral surface of the clutch control ring (60); the alignment teeth (63) of the clutch control ring (60) are able to engage with the alignment grooves (24) in the front end of the tube (20);
multiple single oblique ratchet teeth (74) are arranged around an inner peripheral surface of the connecting component (70); a front end of the clutch control ring (60) rotatably mounted in the connecting component (70); the elastic buckles (62) of the clutch control ring (60) engage with the single oblique ratchet teeth (74) to limit the clutch control ring (60) to be rotated reversely at the predetermined point.

7. The injection mechanism (1) capable of adjusting injection dose as claimed in claim 1, wherein, an abutment ring (41) is mounted to a front end of the elastic assembly (40); the abutment ring (41) is sleeved on the injection push rod (80); the elastic assembly (40) is connected to a rear end of the driving ring (50) via the abutment ring (41).

8. The injection mechanism (1) capable of adjusting injection dose as claimed in claim 7, wherein the elastic assembly (40) is a compression spring; when the tube (20) is rotated by the dose adjustment knob (30), the elastic assembly (40) is driven by the driving ring (50) to be compressed via the abutment ring (41) to store the elastic potential energy.

9. The injection mechanism (1) capable of adjusting injection dose as claimed in any one of claims 1 to 8, wherein, multiple housing-wall-engaging teeth (16) are formed to and arranged around an inner peripheral surface of the rear end of the housing (10) at equiangular intervals;
the tube (20) comprises multiple toothed portions (22) extending backward at an outer peripheral surface of the rear end of the tube (20) and at least one guiding block (23) in front of the toothed portions (22); said guiding block (23) is able to engage with the housing-wall-engaging teeth (16) in the rear end of the housing (10);
the dose adjustment knob (30) comprises an inner annular board (31) in the dose adjustment knob (30) and multiple clutch teeth (32) arranged around a front surface of the inner annular board (31); the clutch teeth (32) of the dose adjustment knob (30) are able to engage with the toothed portions (22) of the tube (20).

10. A syringe capable of adjusting injection dose comprising:
an injection mechanism (1) capable of adjusting injection dose as claimed in any one of claims 1 to 9; and
a cartridge sleeve (3) sleeved on said medicine container (2) and attached to the front end of the housing (10).

11. The syringe capable of adjusting injection dose as claimed in claim 10, wherein at least one cartridge window (33) is formed in a peripheral wall of the cartridge sleeve (3), a status of the liquid medicine in the medicine container (2) is observable through the cartridge window (33); the cartridge sleeve (3) comprises a needle-connecting-end portion (31) at a front end of the cartridge sleeve (3) attachable to a needle (5); and the needle (5) extends through the front end of the cartridge sleeve (3) and is inserted into the medicine container (2).

12. The syringe capable of adjusting injection dose as claimed in claim 10 or 11, wherein
a front-end-connecting portion (14) of the housing (10) has multiple annular grooves (141) spaced apart from one another in a front-to-back arrangement and multiple notches (142) being parallel to a central axis (L) of the injection mechanism (1) formed in an inner peripheral surface of a housing wall (11) of the housing (10);
the cartridge sleeve (3) has an attached-end portion (32) formed at a rear end of the cartridge sleeve (3); an outer peripheral surface of the attached-end portion (32) corresponds to the inner peripheral surface of the housing wall (11) at the front-end-connecting portion (14) of the housing (10) in shape, the attached-end portion (32) at the rear end of the cartridge sleeve (3) is detachably mounted in the front-end-connecting portion (14) of the housing (10).
